# EUROPÄISCHE PATENTSCHRIFT

(11) **EP 3 530 241 B1**
(45) Veröffentlichungstag und Bekanntmachung des Hinweises auf die Patenterteilung: **12.08.2020**
(21) Anmeldenummer: 19158185.9
(22) Anmeldetag: 20.02.2019
(51) Int. Cl.: A61F 5/055

(54) **ZERVIKAL-ORTHESE MIT EINEM U-FÖRMIGEN RAHMEN**
CERVICAL ORTHOTIC WITH A U-SHAPED FRAME
ORTHÈSE CERVICALE DOTÉE D'UN CADRE EN U

(30) Priorität: 22.02.2018 DE 202018100997 U
(43) Veröffentlichungstag der Anmeldung: 28.08.2019
(73) Patentinhaber: Thuasne Deutschland GmbH, 30938 Burgwedel (DE)
(72) Erfinder: Schneider-Nieskens, Reinhold, 29223 Celle (DE); Fritzsche, Stefan, 27308 Kirchlinteln (DE)
(74) Vertreter: Jabbusch, Matthias

(56) Entgegenhaltungen:
- US-A- 5 688 229
- US-A1- 2014 012 172
- US-A1- 2018 000 625
- US-B1- 7 608 052

## Beschreibung

Die Erfindung betrifft eine Zervikal-Orthese mit einem U-förmigen Rahmen, der dazu vorgesehen und ausgelegt ist, auf der Brust eines Patienten aufzuliegen, mit einer Kinnstütze, die U-förmig ausgebildet ist und an ihren beiden Endbereichen über eine Schwenkverbindung mit den Endbereichen des U-förmigen Rahmens verbunden ist, und mit einer Hinterkopfabstützung, die mit dem U-förmigen Rahmen verbunden ist oder verbindbar ist, um zusammen mit dem U-förmigen Rahmen die Zervikal-Orthese ringartig zu schließen, wobei der Rahmen und die Kinnstütze vor der Schwenkverbindung über eine höhenverstellbare Feststelleinrichtung miteinander verbunden sind.

Eine gattungsgemäße Zervikal-Orthese ist beispielsweise aus der US 9,717,621 B2 bekannt. Die Kinnstütze ist dabei schwenkbar mit dem U-förmigen Rahmen verbunden. Die beiden äußeren Seiten der Kinnstütze können dabei unabhängig voneinander mit einer höhenverstellbaren Feststelleinrichtung bewegt werden. Dazu ist in dem Rahmen eine gebogene Führungsbahn ausgebildet, die beidseitig nach innen gerichtete Rippen aufweist und in der ein mit der Kinnstütze verbundener Rastpin auf und ab bewegbar ist. Wird dieser herausgezogen, so kann die Kinnstütze bewegt werden. Wird der Rastpin hereingedrückt, dann ist die Kinnstütze arretiert. 11

Eine gattungsgemäße Zervikal-Orthese gemäss des Oberbegriffs des Schutzsanspruchs 1 ist aus der US 2014/0012172 A1 bekannt. Bei dieser bekannten Zervikal-Orthese ist die höhenverstellbare Feststelleinrichtung auf der beweglichen Kinnstütze angeordnet und die Rastschiene mit dem Rahmen fest verbunden.

Der Erfindung liegt die Aufgabe zugrunde, eine Zervikal-Orthese der eingangs genannten Art zu schaffen, die besonders komfortabel bedienbar ist.

Die Lösung dieser Aufgabe erfolgt mit einer Zervikal-Orthese mit den Merkmalen des Schutzanspruchs 1. Vorteilhafte Ausgestaltungen der Erfindung sind in den Unteransprüchen angegeben.

Bei einer Zervikal-Orthese mit einem U-förmigen Rahmen, der dazu vorgesehen und ausgelegt ist, auf der Brust eines Patienten aufzuliegen, mit einer Kinnstütze, die U-förmig ausgebildet und an ihren beiden Endbereichen über eine Schwenkverbindung mit den Endbereichen des U-förmigen Rahmens verbunden ist, und mit einer Hinterkopfabstützung, die mit dem U-förmigen Rahmen verbunden ist oder verbindbar ist, um zusammen mit dem U-förmigen Rahmen die Zervikal-Orthese ringartig zu schließen, wobei der Rahmen und die Kinnstütze vor der Schwenkverbindung über eine höhenverstellbare Feststelleinrichtung miteinander verbunden sind, wobei die höhenverstellbare Feststelleinrichtung mindestens eine Rastschiene mit mindestens zwei Rastöffnungen zur Arretierung der Kinnstütze in mindestens zwei verschiedenen Höhen aufweist, wobei die höhenverstellbare Feststelleinrichtung mindestens einen Feststeller aufweist, der mit einer Druckfeder derart in Wirkverbindung ist, dass der Feststeller in eine der Rastöffnungen der Rastschiene greift und wobei der Feststeller über eine längliche Entriegelungsverbindung mit einem gegen die Druckrichtung der Druckfeder verschiebbaren Entriegelungselement verbunden ist, ist erfindungswesentlich vorgesehen, dass die Rastschiene mit der Kinnstütze fest verbunden ist. Auf diese Weise lässt sich eine besonders komfortabel bedienbare Zervikal-Orthese schaffen, bei der das Entriegelungselement an einem anderen und besser zugänglichen und bedienbaren Punkt angeordnet ist als die Feststelleinrichtung mit der Rastschiene und dem Feststeller selbst, wobei der Feststeller auf dem Rahmen angeordnet ist, statt auf der beweglichen Kinnstütze.

Bei der oben beschriebenen Zervikal-Orthese versteht sich, dass ein U-förmiger Rahmen zwei seitliche Endbereiche hat, die in einem vorderen Teil miteinander verbunden sind. Der vordere geschlossene Teil des U-förmigen Rahmens und auch der U-förmigen Kinnstütze ist nach vorne zur Brust des Benutzers oder Patienten gerichtet. Die offene Seite ist nach hinten, also zum Rücken des Patienten gerichtet. Diese offene Seite wird mit der Hinterkopfabstützung ringartig, jedoch meist nicht kreisförmig, geschlossen, wobei die Hinterkopfabstützung an den Endbereichen des U-förmigen Rahmens angeschlossen und mit diesem verbunden ist oder verbindbar ist. Typischerweise ist diese Verbindung lösbar, so dass der offene U-förmige Rahmen angelegt werden kann und dann zur Benutzung mit der Hinterkopfabstützung geschlossen wird. Der U-förmige Rahmen erstreckt sich dabei typischerweise über die Schulter des Patienten und ist mit seinem vorderen mittleren Bereich unterhalb des Kinns auf der Brust angeordnet. Die darüberliegende U-förmige Kinnstütze hat ebenfalls zwei seitliche Endbereiche, die mit den seitlichen Endbereichen des U-förmigen Rahmens schwenkbar verbunden sind. Dies kann beispielsweise über eine Nietverbindung erfolgen. Der Rahmen und die Kinnstütze sind an ihren Endbereichen über eine Schwenkverbindung miteinander verbunden. Die zweite Verbindung, die über die höhenverstellbare Feststelleinrichtung erfolgt, ist vor dieser Schwenkverbindung angeordnet, wobei unter dem Begriff "vor" eine räumliche Anordnung zu verstehen ist, wobei der Blickpunkt von der Brust oder dem Kinn des Patienten aus auf die Zervikal-Orthese gerichtet ist und insofern die Rastschiene vor der Schwenkverbindung angeordnet ist. "Vorne" ist insofern als brust- oder kinnseitig, "hinten" als rückenseitig zu verstehen.

Bei der Erfindung ist die Rastschiene fest mit der Kinnstütze verbunden. In einer bevorzugten Ausführungsform ist die Rastschiene einstückig mit der Kinnstütze ausgebildet. Bevorzugt ist die Kinnstütze derart ausgebildet, dass die Rastschiene den U-förmigen Rahmen nach außen übergreift und daher auf der Außenseite des U-förmigen Rahmens anliegt. Gleichzeitig ist der übrige seitliche Endbereich der Kinnstütze flächig ausgebildet und liegt mit seiner Fläche innenseitig an dem U-förmigen Rahmen an, so dass der flächige Endbereich der Kinnstütze und die Rastschiene der Kinnstütze den U-förmigen Rahmen auf beiden Seiten umgreifen und dadurch von diesem geführt werden.

In einer anderen bevorzugten Ausführungsform weist die Kinnstütze zwei schwenkbare Seitenteile und im zentralen Bereich, also im vorderen kinn- oder brustseitigen Bereich, eine die beiden schwenkbaren Seitenteile verbindende Kinnauflage auf, die ihrerseits schwenkbar an den Seitenteilen befestigt ist. Die Kinnauflage ist bevorzugt über je eine Niete an den beiden Seitenteilen schwenkbar befestigt. Dadurch ist eine besonders bequeme Ablage des Kinns auf der Kinnauflage möglich.

Dabei ist die Rastschiene bevorzugt mit den schwenkbaren Seitenteilen der Kinnstütze verbunden oder einstückig mit diesen ausgebildet. Bevorzugt ist dabei die Rastschiene an dem schwenkbaren Seitenteil derart angeordnet, dass sie nah benachbart zu der schwenkbaren Kinnauflage angeordnet ist. Bevorzugt ist die Rastschiene im Bereich der Schwenkverbindung zwischen dem schwenkbaren Seitenteil und der Kinnauflage angeordnet.

In einer weiteren bevorzugten Ausgestaltung der Erfindung ist auf beiden Seiten der Kinnstütze je eine Rastschiene, ein zugeordneter Feststeller und eine dem Feststeller zugeordnete Entriegelungsverbindung mit einem verschiebbaren Entriegelungselement angeordnet. Die beiden Seiten der Kinnstütze lassen sich daher unabhängig voneinander, also auch auf etwas unterschiedliche Höhen mit der höhenverstellbaren Feststelleinrichtung arretieren. Das Entriegelungselement weist dabei bevorzugt ein manuell hin und her bewegbares Betätigungselement auf.

Das Betätigungselement ist dabei bevorzugt konkav, also mit einer nach innen gerichteten Wölbung ausgebildet. Diese ist zu den Endbereichen hin ansteigend, insbesondere in Richtung der Betätigungsrichtung ansteigend. Wenn dort ein Finger eingreift, so stützt er sich innenseitig gegen die ansteigende Wand des gewölbten Betätigungselementes ab. Dadurch ist die Bedienung besonders einfach.

In einer bevorzugten Ausführungsform der Erfindung ist die höhenverstellbare Feststelleinrichtung mit ihren wesentlichen Elementen auf dem Rahmen angeordnet. Dabei ist zumindest auf dem vorderen Teil des Rahmens eine Rahmenabdeckung vorgesehen, die die höhenverstellbare Feststelleinrichtung mit Ausnahme des verschiebbaren Entriegelungselements, insbesondere des Betätigungselements, abdeckt. Dabei ist in der Rahmenabdeckung eine Ausnehmung angeordnet, die derart positioniert ist, dass durch die Ausnehmung das Entriegelungselement betätigbar ist. Insbesondere sind in der Rahmenabdeckung zwei Ausnehmungen angeordnet, so dass dort zwei Entriegelungselemente manuell betätigbar sind, wobei jedes Entriegelungselement einer Rastschiene auf einer der beiden Seiten der Kinnstütze zugeordnet ist.

In einer anderen Weiterbildung der Erfindung ist auf dem U-förmigen Rahmen ein Führungskanal zur Aufnahme der länglichen Entriegelungsverbindung angeordnet. In diesem Führungskanal ist bevorzugt eine Nut oder Quernut zur Aufnahme einer Basis für die Druckfeder vorgesehen. Auf diese Weise lässt sich beispielsweise eine Unterlegscheibe in die Nut in dem Führungskanal ortsfest einlegen. Gegen diese Unterlegscheibe oder Basis stützt sich die Druckfeder ab und kann auf diese Weise eine Vorspannung gegen den Feststeller ausüben, mit der der Feststeller in die entsprechenden Rastöffnungen der Rastschiene eingeschoben wird. Weiterhin ist in dem Führungskanal bevorzugt ein Anschlag ausgebildet, an dem eine korrespondierende geometrische Kontur oder Schulter des Feststellers in der Verriegelungsposition anschlägt.

In einer besonders bevorzugten Ausführungsform ist der Führungskanal nach oben offen ausgebildet, weist also im Wesentlichen Seitenwände auf, die auf dem Rahmen ausgebildet sind, insbesondere einstückig mit dem Rahmen ausgebildet sind. In diesen Führungskanal kann dann die längliche Entriegelungsverbindung und alle weiteren Elemente eingelegt und montiert werden. Bei Montage der Rahmenabdeckung wird der Führungskanal dann von oben geschlossen.

Bei der Erfindung sind die Entriegelungselemente bevorzugt im vorderen mittleren Bereich des Rahmens angeordnet. Auf dem Rahmen sind zwei Führungselemente vorhanden, in denen die Entriegelungselemente geführt sind, wobei die Führungselemente eine Bewegungsrichtung von einer oberen äußeren Position zu einer unteren inneren Position vorgeben. Die Bewegungsrichtung ist dabei derart, dass diese sich unter einem Winkel von ungefähr 90°, also zwischen 80° und 100° schneiden würden. Die beiden Entriegelungselemente sind auch derart im vorderen mittleren Bereich des Rahmens angeordnet, dass eine Betätigung mit Daumen und Zeigefinger einer Hand ohne weiteres möglich ist und eine Bewegung von oben nach unten erlaubt.

Dadurch ist es möglich, beide Entriegelungselemente geleichzeitig zu betätigen. Durch die vorhandenen Rastelemente auf beiden Seiten ist es möglich, die Kinnstütze beliebig einzustellen. Dabei können beide Seiten gleichmäßig verändert werden oder es kann auch eine Seite stärker als die andere Seite verstellt werden. Es ist auch möglich, nur eine Seite der höhenverstellbaren Feststelleinrichtung zu betätigen und dadurch auch nur eine Seite zu verändern und auch insofern individuell eine "schiefe" Einstellung der Kinnstütze vorzunehmen.

Nachfolgend wird die Erfindung anhand eines in der Zeichnung dargestellten Ausführungsbeispiels weiter erläutert. Im Einzelnen zeigen die schematischen Darstellungen in:
- Figur 1:: eine perspektivische Ansicht einer erfindungsgemäßen Zervikal-Orthese;
- Figur 2:: eine perspektivische Ansicht der erfindungsgemäßen Zervikal-Orthese gemäß Figur 1 in einer Explosionsdarstellung;
- Figur 3:: einen vergrößerten Ausschnitt der höhenverstellbaren Feststelleinrichtung;
- Figur 4:: ein Detail der höhenverstellbaren Feststelleinrichtung;
- Figur 5:: eine perspektivische Ansicht der Zervikal-Orthese ohne die Rahmenabdeckung und mit der Kinnstütze in zwei verschiedenen Positionen; und
- Figur 6:: eine Frontansicht der Zervikal-Orthese mit angedeuteter Bewegungsrichtung der Betätigungselemente.

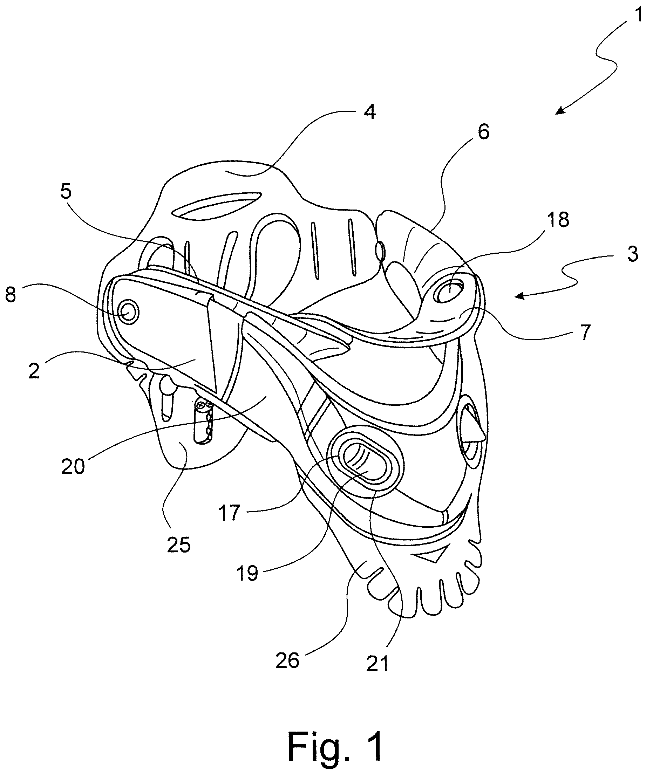

In Figur 1 ist die Zervikal-Orthese 1, die auch als Halskrause bezeichnet wird, in einer perspektivischen Ansicht dargestellt. Die Zervikal-Orthese 1 weist die Hauptelemente mit einem Rahmen 2, einer Kinnstütze 3 und einer Hinterkopfabstützung 4 auf. Die Zervikal-Orthese 1 ist im Wesentlichen aus Kunststoff hergestellt. Der Rahmen 2 ist im Wesentlichen U-förmig ausgebildet, hat also zwei seitliche Endbereiche, die auf der Schulter des Patienten aufliegen und einen vorderen Bereich, der auf der Brust des Patienten zu liegen kommt und eine untere vordere Verlängerung 26 aufweist, die für eine besonders gute Brustauflage sorgt. Um die Zervikal-Orthese 1 nach hinten zu schließen, ist dem U-förmigen Rahmen 2 eine Hinterkopfabstützung 4 zugeordnet, die an die beiden Endbereiche des U-förmigen Rahmens 2 anschließbar und mit diesen verbindbar ist. Die Hinterkopfabstützung 4 weist nach unten eine Verlängerung auf, die als längenjustierbare Rückenauflage 25 ausgebildet ist. Der Rahmen 2 weist in seinem vorderen Bereich eine Rahmenabdeckung 20 auf. Unterhalb der Rahmenabdeckung 20 verbirgt sich ein wesentlicher Teil der höhenverstellbaren Feststelleinrichtung, mit der die Kinnstütze 3 gegenüber dem Rahmen 2 höhenverstellbar ist. Die Kinnstütze 3 weist im Wesentlichen schwenkbare Seitenteile 5, 6 und eine zentrale Kinnauflage 7 auf. Die schwenkbaren Seitenteile 5, 6 sind über Schwenkverbindungen 8, die als Nieten ausgebildet sind, an den Endbereichen des U-förmigen Rahmens 2 schwenkbar befestigt. Die Kinnauflage 7 ist über Schwenkverbindungen 18, die ebenfalls als Nieten ausgebildet sind, schwenkbar mit den schwenkbaren Seitenteilen 5, 6 verbunden. Im vorderen zentralen Bereich der Zervikal-Orthese 1 und insbesondere der Rahmenabdeckung 20 sind manuell betätigbare Betätigungselemente 19 zu sehen, die auf beiden Seiten angeordnet sind. Die manuell betätigbaren Betätigungselemente 19 sind Teil eines Entriegelungselementes 17, das in Ausnehmungen 21 der Rahmenabdeckung 20 verschiebbar sind.

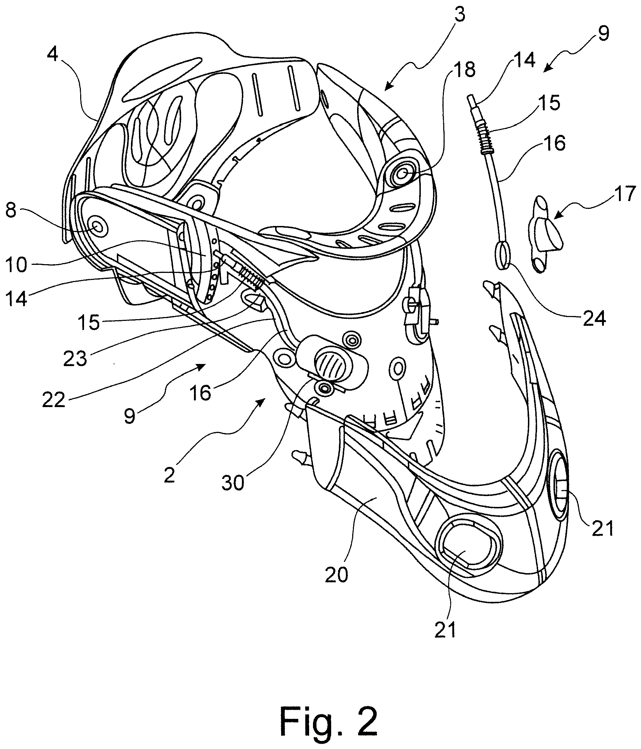

In Figur 2 ist die erfindungsgemäße Zervikal-Orthese 1 gemäß Figur 1 in einer Explosionsdarstellung dargestellt. Gleiche Teile sind mit gleichen Bezugszahlen versehen. Insbesondere ist hier die Rahmenabdeckung 20 entfernt worden, so dass der untere Teil des Rahmens 2 sichtbar wird, auf dem die höhenverstellbare Feststelleinrichtung 9 angeordnet ist. Diese weist eine Rastschiene 10 auf, die Teil der Kinnstütze 3 und insbesondere Teil der schwenkbaren Seitenteile 5, 6 der Kinnstütze 3 ist. Die Rastschiene 10 erstreckt sich ausgehend von dem schwenkbaren Seitenteil 5 nach unten und weist eine Mehrzahl von nach vorne gerichteten Rastöffnungen 11, 12, 13 auf. Je nach dem, in welcher dieser Rastöffnungen 11, 12, 13 oder weiterer Rastöffnungen die höhenverstellbare Feststelleinrichtung 9 arretiert wird, so ist die Höhe der schwenkbaren Kinnstütze 3 eingestellt. In die entsprechende Rastöffnung 11, 12, 13 greift ein Feststeller 14 ein, der mit einer Druckfeder 15 vorbelastet ist und daher zuverlässig in die entsprechende Rastöffnung eingreift und die Höhe dadurch vorgibt. Der Feststeller 14 ist in einem Führungskanal 22 angeordnet und darin positioniert. In dem Führungskanal 22 ist auch eine an den Feststeller 14 angeschlossene Entriegelungsverbindung 16 angeordnet, die als flexible längliche Verbindung ausgelegt ist. Über ein Entriegelungselement 17 kann ein Zug über die Entriegelungsverbindung 16 gegen die Druckrichtung der Druckfeder 15 auf den Feststeller 14 ausgeübt werden und der Feststeller 14 aus der entsprechenden Rastöffnung 11, 12, 13 herausgezogen werden. Dann ist die Kinnstütze 3 in ihrer Höhe verschiebbar. Bei Freigabe des Entriegelungselements 17 wird der Feststeller 14 durch die Druckfeder 15 wieder in die dann vorgegebene Rastöffnung 11, 12, 13 einrasten und die Kinnstütze 3 in dieser Position feststellen. Die Entriegelungsverbindung 16 weist an ihrem Ende ein Verbindungselement 24 auf, das bevorzugt als geschlossener Kreis ausgebildet ist und mit einem korrespondierenden Ring an der Unterseite des Entriegelungselements 17 korrespondiert und auf diese Weise mit dem Entriegelungselement 17 verbunden ist.

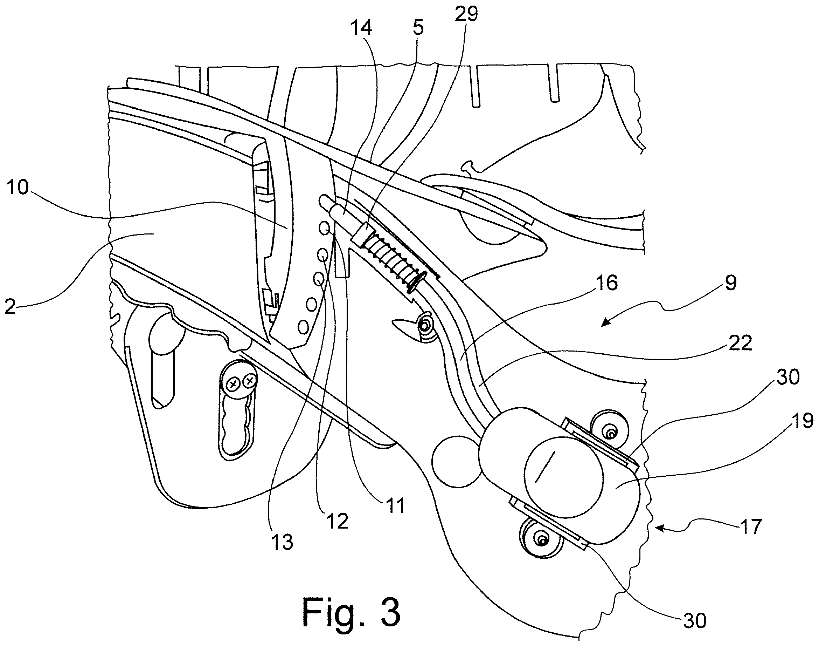

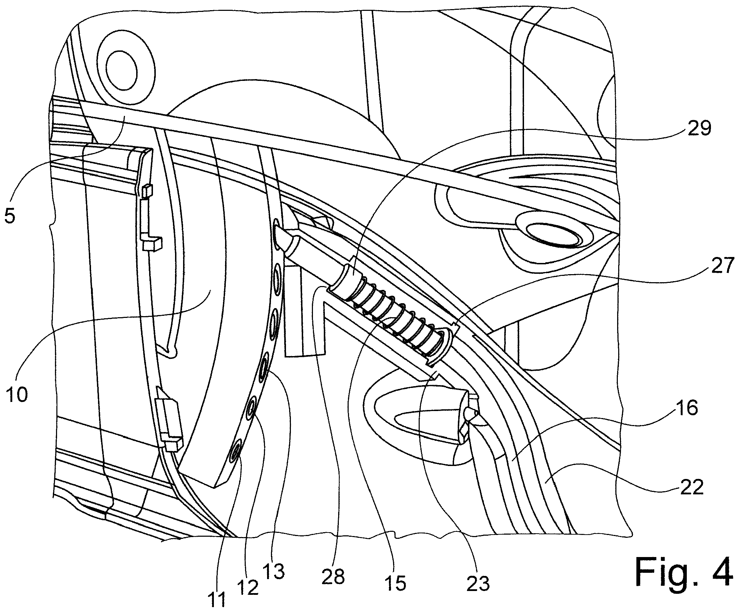

In Figur 3 ist ein Ausschnitt der erfindungsgemäßen Zervikal-Orthese 1 mit der höhenverstellbaren Feststelleinrichtung 9 dargestellt. In Figur 4 ist dieser Ausschnitt im Bereich der Rastschiene 10 mit dem Feststeller 14 weiter vergrößert. Die Figuren 3 und 4 werden im Folgenden gemeinsam beschrieben. Auf dem Rahmen 2 ist auf seiner Außenseite die Rastschiene 10 positioniert. Diese greift an der Oberkante der Kinnstütze 3, insbesondere des schwenkbaren Seitenteils 5 an, das im Übrigen auf der Innenseite des Rahmens liegt und auf diese Weise den Rahmen 2 übergreift. In der Rastschiene 10 sind hier insgesamt sechs Rastöffnungen vorgesehen, von denen hier drei mit den Bezugszahlen 11, 12 und 13 versehen sind. Im gezeigten Ausführungsbeispiel lässt sich daher die Kinnstütze 3 in sechs verschiedenen Höhenstufen arretieren. Dazu wird die Kinnstütze 3 bei nach vorne gezogenem Entriegelungselement 17 in die gewünschte Höhe gebracht und dann wird das Entriegelungselement 17 freigegeben. Dadurch wird der Feststeller 14 von der Druckfeder 15 in eine der Rastöffnungen 11, 12, 13 gedrückt. Der Feststeller 14 ist über die längliche flexible Entriegelungsverbindung 16 mit dem Entriegelungselement 17 verbunden. Die Entriegelungsverbindung 16 verläuft in dem Führungskanal 22. Dieser weist in seinem vorderen Bereich eine besonders geformte Aufnahme für die Druckfeder 15 und den Feststeller 14 auf. Insbesondere ist quer zur Längsachse des Führungskanals 22 eine Nut 27 vorgesehen, in der eine Unterlegscheibe oder Basis 23 angeordnet ist, an der sich die Druckfeder 15 abstützt. Nach vorne stößt die Druckfeder 15 gegen eine entsprechende Aufnahme in dem Feststeller 14. Der Feststeller 14 hat zudem an seiner Vorderkante eine Schulter 29, die an einem korrespondierenden Anschlag 28 in dem Führungskanal 22 anschlägt.

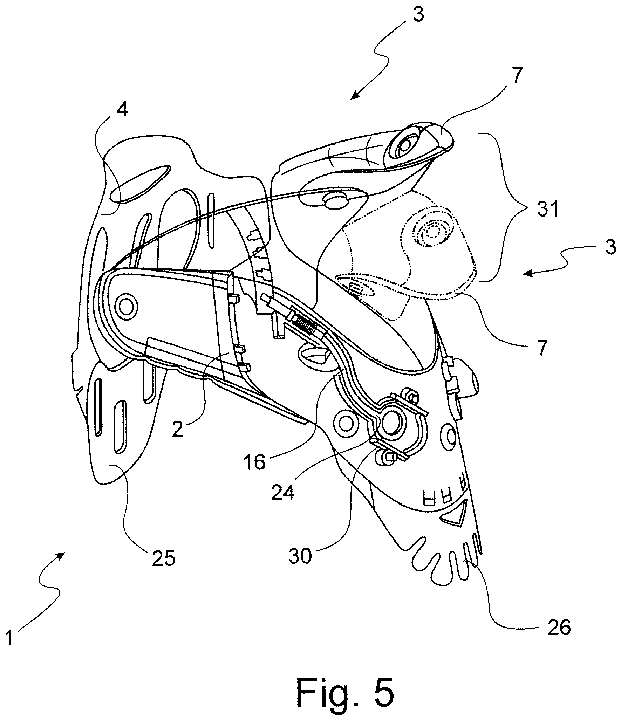

In Figur 5 ist die erfindungsgemäße Zervikal-Orthese 1 in einer perspektivischen Ansicht vergleichbar zu den Figuren 1 und 2 dargestellt. Die Rahmenabdeckung 20 ist entfernt. Wesentlich ist hier, dass die Kinnstütze 3 in verschiedenen Positionen dargestellt ist. Zwischen diesen beiden Positionen ergibt sich eine Höhendifferenz 31 von mehreren Zentimetern, insbesondere von fünf bis zehn Zentimetern, so dass eine individuelle Einstellung der Zervikal-Orthese 1 mit einer individuell einstellbaren Kinnstütze 3 möglich ist. Im Übrigen sind gleiche Teile mit gleichen Bezugszeichen benannt.

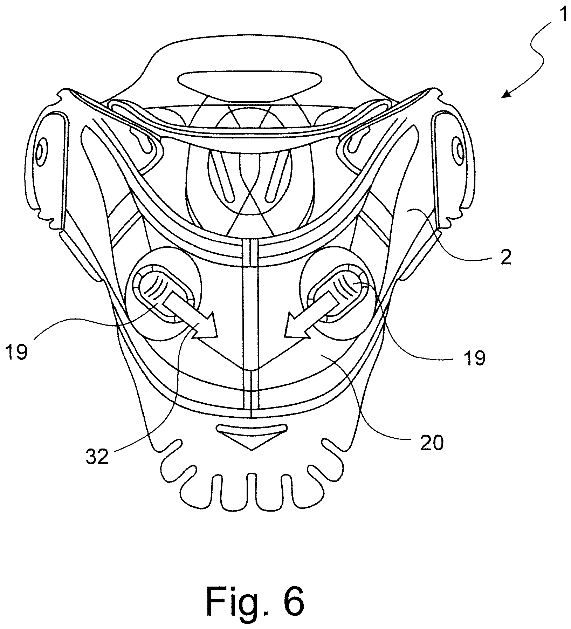

In Figur 6 ist eine Frontansicht der erfindungsgemäßen Zervikal-Orthese 1 dargestellt. Wesentlich ist hier, dass die Entriegelungselemente 17 zur Entriegelung der höhenverstellbaren Feststelleinrichtung 9 durch die Betätigungselemente 19 bedient werden. Diese sind geriffelt und dadurch manuell leicht bedienbar. Knopfelemente ausgebildet. Die Entriegelungselemente 17 und damit auch die Betätigungselemente 19 sind nebeneinander im vorderen zentralen Teil der Zervikal-Orthese 1 angeordnet. Die durch die Führungselemente 30 vorgegebene Bewegungsrichtung ist schräg nach unten. Die Betätigungsrichtungen 32 der beiden Elemente treffen in einem Winkel zwischen 70° und 100°, insbesondere zwischen 85° und 95° aufeinander. Die beiden Betätigungselemente 19 sind typischerweise so nah zueinander benachbart, dass die beiden Betätigungselemente 19 bequem mit Daumen und Zeigefinger einer Hand betätigbar sind. Dadurch ist eine besonders einfache Bedienung möglich.

Alle in der vorstehenden Beschreibung und in den Ansprüchen genannten Merkmale sind in einer beliebigen Auswahl mit den Merkmalen des unabhängigen Anspruchs kombinierbar. Die Offenbarung der Erfindung ist somit nicht auf die beschriebenen bzw. beanspruchten Merkmalskombinationen beschränkt, vielmehr sind alle im Rahmen der Erfindung sinnvollen Merkmalskombinationen als offenbart zu betrachten.

## Patentansprüche

1. Zervikal-Orthese mit einem U-förmigen Rahmen (2), der dazu vorgesehen und ausgelegt ist, auf der Brust eines Patienten aufzuliegen, mit einer Kinnstütze (3), die U-förmig ausgebildet ist und an ihren beiden Endbereichen über eine Schwenkverbindung mit den Endbereichen des U-förmigen Rahmens (2) verbunden ist, und mit einer Hinterkopfabstützung (4), die mit dem U-förmigen Rahmen (2) verbunden ist oder verbindbar ist, um zusammen mit dem U-förmigen Rahmen (2) die Zervikal-Orthese ringartig zu schließen, wobei der Rahmen und die Kinnstütze (3) vor der Schwenkverbindung über eine höhenverstellbare Feststelleinrichtung (9) miteinander verbunden sind,
wobei die höhenverstellbare Feststelleinrichtung (9) mindestens eine Rastschiene (10) mit mindestens zwei Rastöffnungen (11, 12, 13) zur Arretierung der Kinnstütze (3) in mindestens zwei verschiedenen Höhen aufweist,
wobei die höhenverstellbare Feststelleinrichtung (9) mindestens einen Feststeller (14) aufweist, der mit einer Druckfeder (15) derart in Wirkverbindung ist, dass der Feststeller (14) in eine der Rastöffnungen (11, 12, 13) der Rastschiene (10) eingreift,
und wobei der Feststeller (14) über eine längliche Entriegelungsverbindung (16) mit einem gegen die Druckrichtung der Druckfeder (15) verschiebbaren Entriegelungselement (17) verbunden ist,
**dadurch gekennzeichnet,**
**dass** die Rastschiene (10) mit der Kinnstütze (3) fest verbunden ist.

2. Zervikal-Orthese nach Anspruch 1, **dadurch gekennzeichnet, dass** die Rastschiene (10) einstückig mit der Kinnstütze (3) verbunden ist.

3. Zervikal-Orthese nach Anspruch 1 oder 2, **dadurch gekennzeichnet, dass** die Kinnstütze (3) zwei schwenkbare Seitenteile (5, 6) und im zentralen vorderen Bereich eine die beiden schwenkbaren Seitenteile (5, 6) verbindende Kinnauflage (7) aufweist, die ihrerseits schwenkbar an den Seitenteilen (5, 6) befestigt ist.

4. Zervikal-Orthese nach einem der Ansprüche 1 bis 3, **dadurch gekennzeichnet, dass** die Rastschiene (10) mit den schwenkbaren Seitenteilen (5, 6) der Kinnstütze (3) verbunden ist.

5. Zervikal-Orthese nach Anspruch 4, **dadurch gekennzeichnet, dass** die Rastschiene (10) an dem schwenkbaren Seitenteil (5, 6) nah benachbart zu der schwenkbaren Kinnauflage (7) angeordnet ist.

6. Zervikal-Orthese nach einem der vorhergehenden Ansprüche, **dadurch gekennzeichnet, dass** auf beiden Seiten der Kinnstütze (3) je eine Rastschiene (10), ein zugeordneter Feststeller (14) und eine dem Feststeller (14) zugeordnete Entriegelungsverbindung (16) mit einem verschiebbaren Entriegelungselement (17) angeordnet ist.

7. Zervikal-Orthese nach einem der vorhergehenden Ansprüche, **dadurch gekennzeichnet, dass** das Entriegelungselement (17) ein manuell hin und her bewegbares Betätigungselement (19) aufweist.

8. Zervikal-Orthese nach Anspruch 7, **dadurch gekennzeichnet, dass** das Betätigungselement (19) eine konkave, nach innen gewölbte Form aufweist, die in Auslöserichtung ansteigend ist.

9. Zervikal-Orthese nach einem der vorhergehenden Ansprüche, **dadurch gekennzeichnet, dass** die höhenverstellbare Feststelleinrichtung (9) auf dem Rahmen (2) angeordnet ist und dass zumindest auf dem vorderen Teil des Rahmens (2) eine Rahmenabdeckung (20) vorgesehen ist, die die höhenverstellbare Feststelleinrichtung (9) mit Ausnahme des verschiebbaren Entriegelungselements (17) abdeckt, wobei in der Rahmenabdeckung (20) eine Ausnehmung (21) angeordnet ist, die derart positioniert ist, dass durch die Ausnehmung (21) das Entriegelungselement (17) betätigbar ist.

10. Zervikal-Orthese nach einem der vorhergehenden Ansprüche, **dadurch gekennzeichnet, dass** auf dem U-förmigen Rahmen (2) ein Führungskanal (22) zur Aufnahme der länglichen Entriegelungsverbindung (16) aufweist.

11. Zervikal-Orthese nach Anspruch 10, **dadurch gekennzeichnet, dass** in dem Führungskanal (22) eine Nut (27) zur Aufnahme einer Basis (23) für die Druckfeder (15) vorgesehen ist.

12. Zervikal-Orthese nach einem der Ansprüche 10 oder 11, **dadurch gekennzeichnet, dass** in dem Führungskanal (22) ein Anschlag (28) ausgebildet ist, an dem eine korrespondierende geometrische Kontur des Feststellers (14) in der Verriegelungsposition anschlägt.

13. Zervikal-Orthese nach einem der Ansprüche 10 bis 12, **dadurch gekennzeichnet, dass** der Führungskanal (22) nach oben offen ist und von oben durch die Rahmenabdeckung (20) abgedeckt ist.

14. Zervikal-Orthese nach einem der vorhergehenden Ansprüche, **dadurch gekennzeichnet, dass** zwei Entriegelungselemente (17) im vorderen mittleren Bereich des Rahmens (2) angeordnet sind.

15. Zervikal-Orthese nach Anspruch 14, **dadurch gekennzeichnet, dass** die Entriegelungselemente (17) so nah benachbart sind, dass diese gleichzeitig mit zwei Fingern einer Hand betätigbar sind.

16. Zervikal-Orthese nach einem der vorhergehenden Ansprüche, **dadurch gekennzeichnet, dass** auf dem Rahmen (2) zwei Führungselemente vorhanden sind, in denen die Entriegelungselemente (17) geführt werden, wobei die Führungselemente eine Bewegungsrichtung von einer oberen äußeren Position zu einer unteren inneren Position vorgeben.

17. Zervikal-Orthese nach einem der vorhergehenden Ansprüche, **dadurch gekennzeichnet, dass** die Kinnstütze (3) auf beiden Seiten unabhängig voneinander verstellbar ist.

## Claims

1. A cervical orthotic with a U-shaped frame (2), which is intended and designed to rest on a patient's chest, with a chin rest (3) which is U-shaped and at its two end regions is connected via a pivot connection to the end regions of the U-shaped frame (2), and with a support (4) for the back of the head, which support is connected or is connectable to the U-shaped frame (2) in order to, together with the U-shaped frame (2), close the cervical orthotic in a ring-like fashion, the frame and the chin rest (3) being connected to one another in front of the pivot connection via a height-adjustable fixing device (9),
the height-adjustable fixing device (9) having at least one catch rail (10) with at least two catch openings (11, 12, 13) for locking the chin rest (3) at least at two different heights,
the height-adjustable fixing device (9) having at least one retainer (14), which is operatively connected to a compression spring (15) in such a way that the retainer (14) engages in one of the catch openings (11, 12, 13) in the catch rail (10), and
the retainer (14) being connected via an elongate unlocking connection (16) to an unlocking element (17) displaceable against the pressure direction of the compression spring (15),
**characterised in that**
the catch rail (10) is fixedly connected to the chin rest (3).

2. The cervical orthotic according to claim 1, **characterised in that** the catch rail (10) is integrally connected to the chin rest (3).

3. The cervical orthotic according to claim 1 or 2, **characterised in that** the chin rest (3) has two pivotable side parts (5, 6) and in the central front region has a chin support (7) connecting the two pivotable side parts (5, 6), which chin support is in turn fastened pivotably to the side parts (5, 6).

4. The cervical orthotic according to any one of claims 1 to 3, **characterised in that** the catch rail (10) is connected to the pivotable side parts (5, 6) of the chin rest (3).

5. The cervical orthotic according to claim 4, **characterised in that** the catch rail (10) is arranged on the pivotable side part (5, 6) closely adjacently to the pivotable chin support (7).

6. The cervical orthotic according to any one of the preceding claims, **characterised in that** on each of the two sides of the chin rest (3) there are arranged a catch rail (10), an associated retainer (14), and an unlocking connection (16) associated with the retainer (14), said unlocking connection having a displaceable unlocking element (17).

7. The cervical orthotic according to any one of the preceding claims, **characterised in that** the unlocking element (17) has an actuation element (19) manually movable back and forth.

8. The cervical orthotic according to claim 7, **characterised in that** the actuation element (19) has a concave, inwardly curved form, which ascends in the release direction.

9. The cervical orthotic according to any one of the preceding claims, **characterised in that** the height-adjustable fixing device (9) is arranged on the frame (2), and **in that** a frame cover (20) is provided at least on the front part of the frame (2), which frame cover covers the height-adjustable fixing device (9) with the exception of the displaceable unlocking element (17), a recess (21) being arranged in the frame cover (20), which recess is positioned in such a way that the unlocking element (17) is actuatable through the recess (21).

10. The cervical orthotic according to any one of the preceding claims, **characterised in that** a guide channel (22) for receiving the elongate unlocking connection (16) is provided on the U-shaped frame (2).

11. The cervical orthotic according to claim 10, **characterised in that** a groove (27) for receiving a base (23) for the compression spring (15) is provided in the guide channel (22).

12. The cervical orthotic according to any one of claims 10 or 11, **characterised in that** a stop (28) is formed in the guide channel (22) and a corresponding geometrical contour of the retainer (14) strikes against the stop in the locking position.

13. The cervical orthotic according to any one of claims 10 to 12, **characterised in that** the guide channel (22) is upwardly open and is covered from above by the frame cover (20).

14. The cervical orthotic according to any one of the preceding claims, **characterised in that** two unlocking elements (17) are arranged in the front middle region of the frame (2).

15. The cervical orthotic according to claim 14, **characterised in that** the unlocking elements (17) are arranged adjacently so closely that they are actuatable simultaneously using two fingers of the same hand.

16. The cervical orthotic according to any one of the preceding claims, **characterised in that** two guide elements are provided on the frame (2), the unlocking elements (17) being guided in said guide elements and the guide elements predefining a movement direction from an upper outer position to a lower inner position.

17. The cervical orthotic according to any one of the preceding claims, **characterised in that** the chin rest (3) is adjustable on both sides independently of one another.

## Revendications

1. Orthèse cervicale dotée d'un cadre en U (2) qui est prévue et conçue pour reposer sur la poitrine d'un patient, comprenant un support de menton (3) qui est formé en U et relié par ses deux parties d'extrémité aux parties d'extrémité du cadre en U (2) par une liaison pivotante, et comprenant un support d'arrière-tête (4) qui est relié ou peut être relié au cadre en U (2) pour fermer l'orthèse cervicale en forme d'anneau conjointement avec le cadre en U (2), dans laquelle le cadre et le support de menton (3) sont reliés entre eux par un dispositif de fixation réglable en hauteur (9) devant la liaison pivotante,
dans laquelle le dispositif de fixation réglable en hauteur (9) présente au moins un rail d'encliquetage (10) comprenant au moins deux ouvertures d'encliquetage (11, 12, 13) pour bloquer le support de menton (3) dans au moins deux hauteurs différentes,
dans laquelle le dispositif de fixation réglable en hauteur (9) présente au moins un fixateur (14) qui est en liaison active avec un ressort de pression (15) de telle sorte que le fixateur (14) se met en prise dans une des ouvertures d'encliquetage (11, 12, 13) du rail d'encliquetage (10), et
dans laquelle le fixateur (14) est relié à un élément de déverrouillage (17) mobile contre le sens de pression du ressort de pression (15) par le biais d'une liaison de déverrouillage (16) oblongue,
**caractérisée en ce que**
le rail d'encliquetage (10) est relié fixement au support de menton (3).

2. Orthèse cervicale selon la revendication 1, **caractérisée en ce que** le rail d'encliquetage (10) est relié d'une seule pièce au support de menton (3).

3. Orthèse cervicale selon la revendication 1 ou 2, **caractérisée en ce que** le support de menton (3) présente deux parties latérales (5, 6) pivotantes et dans la partie centrale avant, un appui de menton (7) reliant les deux parties latérales (5, 6) pivotantes, qui à son tour est fixé pivotant aux parties latérales (5, 6).

4. Orthèse cervicale selon l'une des revendications 1 à 3, **caractérisée en ce que** le rail d'encliquetage (10) est relié aux parties latérales (5, 6) pivotantes du support de menton (3).

5. Orthèse cervicale selon la revendication 4, **caractérisée en ce que** le rail d'encliquetage (10) sur la partie latérale (5, 6) pivotante est disposé à proximité proche de l'appui de menton (7) pivotant.

6. Orthèse cervicale selon l'une des revendications précédentes, **caractérisée en ce que** respectivement un rail d'encliquetage (10), un fixateur (14) correspondant et un dispositif de déverrouillage (16) correspondant au fixateur (14), comprenant un élément de déverrouillage (17) mobile, est disposé sur les deux côtés du support de menton (3).

7. Orthèse cervicale selon l'une des revendications précédentes, **caractérisée en ce que** l'élément de déverrouillage (17) présente un élément d'actionnement (19) pouvant être actionné d'avant en arrière.

8. Orthèse cervicale selon la revendication 7, **caractérisée en ce que** l'élément d'actionnement (19) présente une forme concave bombée vers l'intérieur, qui augmente dans le sens de séparation.

9. Orthèse cervicale selon l'une des revendications précédentes, **caractérisée en ce que** le dispositif de fixation réglable en hauteur (9) est disposé sur le cadre (2) et qu'au moins sur la partie avant du cadre (2), il est prévu un couvercle de cadre (20) qui recouvre le dispositif de fixation réglable en hauteur (9) à l'exception de l'élément de déverrouillage (17) mobile, dans laquelle un évidement (21) est prévu dans le couvercle de cadre (20), qui est positionné de façon à ce que l'élément de déverrouillage (17) puisse être actionné par l'évidement (21).

10. Orthèse cervicale selon l'une des revendications précédentes, **caractérisée en ce qu'**un canal de guidage (22) est présent sur le cadre en U (2) pour recevoir la liaison de déverrouillage (16) oblongue.

11. Orthèse cervicale selon la revendication 10, **caractérisée en ce qu'**une rainure (27) pour recevoir une base (23) pour le ressort de pression (15) est prévue dans le canal de guidage (22).

12. Orthèse cervicale selon l'une des revendications 10 ou 11, **caractérisée en ce qu'**une butée (28) est conçue dans le canal de guidage (22) sur laquelle vient buter un contour géométrique correspondant du fixateur (14) dans la position de verrouillage.

13. Orthèse cervicale selon l'une des revendications 10 à 12, **caractérisée en ce que** le canal de guidage (22) est ouvert vers le haut et couvert par le haut par le couvercle de cadre (20).

14. Orthèse cervicale selon l'une des revendications précédentes, **caractérisée en ce que** deux éléments de déverrouillage (17) sont prévus dans la partie centrale avant du cadre (2).

15. Orthèse cervicale selon la revendication 14, **caractérisée en ce que** les éléments de déverrouillage (17) sont si près l'un de l'autre que ceux-ci peuvent être actionnés en même temps par deux doigts d'une main.

16. Orthèse cervicale selon l'une des revendications précédentes, **caractérisée en ce que** deux éléments de guidage sont présents sur le cadre (2), dans lesquels les éléments de déverrouillage (17) sont guidés, dans laquelle les éléments de guidage prédéfinissent un sens de déplacement d'une position extérieure supérieure à une position intérieure inférieure.

17. Orthèse cervicale selon l'une des revendications précédentes, **caractérisée en ce que** le support de menton (3) est réglable sur les deux côtés indépendamment l'un de l'autre.
